# EUROPEAN PATENT APPLICATION

(11) **EP 2 728 326 A1**
(43) Date of publication of application: **07.05.2014**
(21) Application number: 11858472.1
(22) Date of filing: 08.08.2011
(51) Int. Cl.: G01K 1/00, G01J 5/00, A61B 1/227, A61B 1/24, A61B 5/00

(54) **PROBE STRUCTURE, TEMPERATURE MEASURING DEVICE HAVING SAME AND METHOD FOR USING SAME**

(30) Priority: 30.06.2011 CN 201110185500
(71) Applicant: Microlife Intellectual Property GmbH, Widnau 9443 St. Gallen (CH)
(72) Inventor: HO, Chia-chen, Taipei City 114 Taiwan (TW)
(74) Representative: Viering, Jentschura & Partner Patent- und Rechtsanwälte
(86) International application number: PCT/CN2011/078098
(87) International publication number: WO 2013/000191

(57) **Abstract**

The present invention relates to a probe structure, a temperature measuring device having the probe structure, and a method of using the same. The probe structure comprises a light transmissive unit and one or more light sources. One or more light sources radiate one or more colors of light to and through the light transmissive unit. The temperature measuring device includes the above-mentioned probe structure and a temperature measuring body. The probe structure is assembled within the temperature measuring body. By the design of the light transmissive unit and the visual light provided by the light sources, the user can easily place the probe structure to the part to be measured.

## Description

### FIELD OF THE INVENTION

This invention relates to a probe structure, temperature measuring device having the probe structure, and method of using the temperature measuring device.

### BACKGROUND OF THE INVENTION

Conventionally, a user needs to put a probe structure of a temperature measuring device, such as ear thermometer or body thermometer into ear or mouth of a subject to proceed with the measurement of body temperature.

The conventional temperature measuring device, such as U.S. patent publication No. US2005/0080354, only provides light source to let users place the probe structure to the site to be measured. However, it has a problem that the light can be easily blocked.

Moreover, JP patent publication No. 9-5167 discloses an ear thermometer utilizing infrared ray to measure body temperature. After the probe structure of the ear thermometer is inserted into ear, the infrared ray came from eardrum and its peripheral is detected by the infrared ray detector of the ear thermometer. In the mean time, the user can see the eardrum and its peripheral via the window of the ear thermometer and adjust the measure position by the visible light provided by the end of the probe.

However, the visible light disclosed by the above-mentioned conventional technique is used to adjust the measure position after placing the probe structure of the ear thermometer into ear. Therefore, it is not ideal to use the ear thermometer in dark environment.

Further, the user can see the visible light only via the window of the ear thermometer and then see the eardrum and its peripheral, therefore, there should be a plurality of reflection mirrors and light guide plates inside the ear thermometer so that the visible light can be guided to the window for user to see, his design will be very complicated.

### SUMMARY OF THE INVENTION

In order to solve the above and conventional technical problems, the present invention provides a probe structure including a light transmissive unit and one or more light sources. The one or more light sources emit one or more colors of lights to and through the light transmissive unit.

The light sources of the probe structure emit one or more colors of lights so that different conditions can be indicated by changing the colors. For example, a user can directly see the displayed (warning) result from the light through the light transmissive unit of the probe structure when the body temperature is too high or too low.

The present invention further provides a temperature measuring device having a probe structure. The temperature measuring device includes a probe structure and a temperature measuring body. The probe structure includes a light transmissive unit, one or more light sources, and a temperature detecting module. The probe structure is assembled to the temperature measuring body which includes a circuit board, a button and a temperature display. The button is electrically connected to the circuit board and the temperature detecting module of the probe structure is electrically connected to the circuit board as well.

The present invention further provides a method of using the above-mentioned temperature measuring device, wherein the lights of the one or more light sources include a first color of light and a second color of light, and the method includes the steps of: (A) pressing the button of the temperature measuring body; and (B) the one or more light sources emitting the first color of light from the one or more light sources to and through the light transmissive unit.

The present invention further provides a method of using the above-mentioned temperature measuring device, wherein the lights of the one or more light sources include a first color of light and a second color of light, and the method includes the steps of: (A) a user pressing the button of the temperature measuring body; (B) the one or more light sources emitting the first color of light; (C) the first color of light emitting to and through the light transmissive unit; (D) the user searching a site to be measured of a subject by the light emitted from the light transmissive unit; (E) the user positioning the temperature measuring device to the site to be measured by being aware of the position of the light transmissive unit that emits light; (F) determining if a measured temperature measured by the temperature measuring device exceeds a predetermined temperature; and (G) the one or more light sources emitting the second color of light if the measured temperature exceeds the predetermined temperature.

The present invention further provides a method of using the above-mentioned temperature measuring device, wherein the lights of the one or more light sources include a first color of light and a second color of light, and the method includes the steps of: (A) a user pressing the button of the temperature measuring body; (B) the one or more light sources emitting the first color of light; (C) the first color of light emitting to and through the light transmissive unit; (D) the user searching a site to be measured of a subject by the light emitted from the light transmissive unit; (E) the user positioning the temperature measuring device to the site to be measured by being aware of the position of the light transmissive unit that emits light; (F) determining if a measured temperature measured by the temperature measuring device exceeds a predetermined temperature; and (G) the one or more light sources emitting the second color of light if the measured temperature does not exceed the predetermined temperature.

### BRIEF DESCRIPTION OF DRAWINGS

Figure 1 shows a perspective view of a first preferred embodiment of the present invention.
Figure 2 shows an explosion view of the first preferred embodiment of the present invention.
Figure 3 shows a sectional view of the first preferred embodiment of the present invention.
Figure 4 shows a perspective view of a second preferred embodiment of the present invention.
Figure 5 shows a perspective view of a third preferred embodiment of the present invention.
Figure 6 shows an explosion view of a third preferred embodiment of the present invention.
Figure 7 shows a sectional view of a third preferred embodiment of the present invention.
Figure 8 shows a perspective view of a fourth preferred embodiment of the present invention.
Figure 9 shows a partial sectional view of a fifth preferred embodiment of the present invention.
Figure 10 shows a sectional view of a sixth preferred embodiment of the present invention.
Figure 11 shows a flow chart of a seventh preferred embodiment of the present invention.
Figure 12 shows a flow chart of an eighth preferred embodiment of the present invention.
Figure 13 shows a flow chart of a ninth preferred embodiment of the present invention.

### DETAILED DESCRIPTION OF SPECIFIC EMBODIMENTS

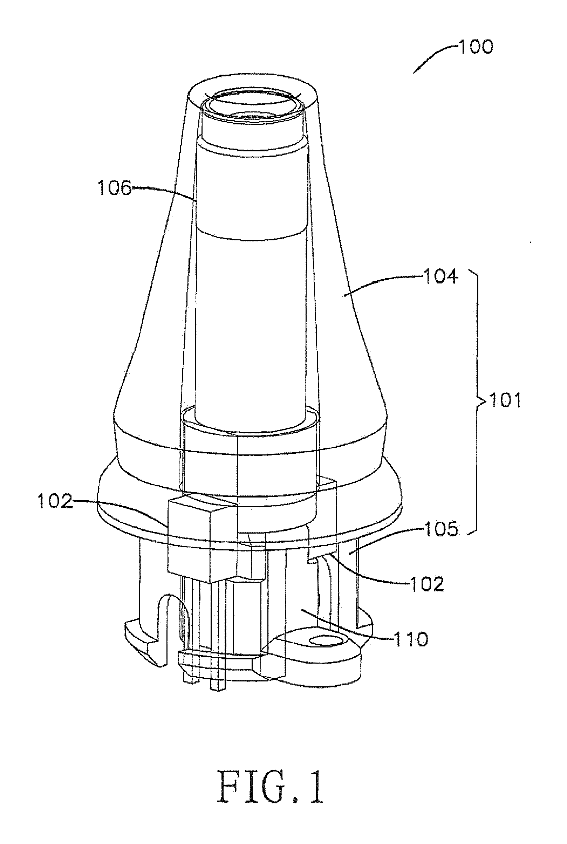

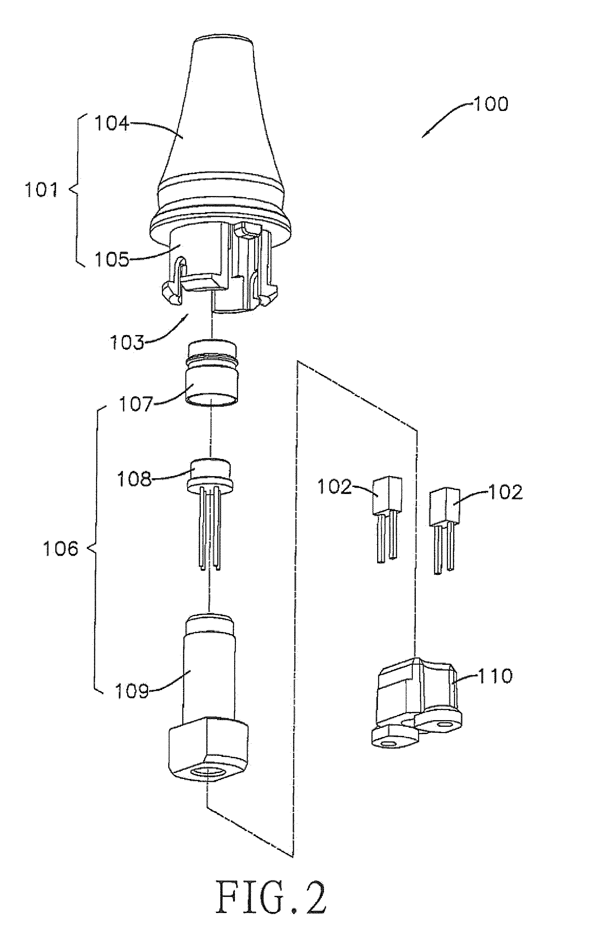

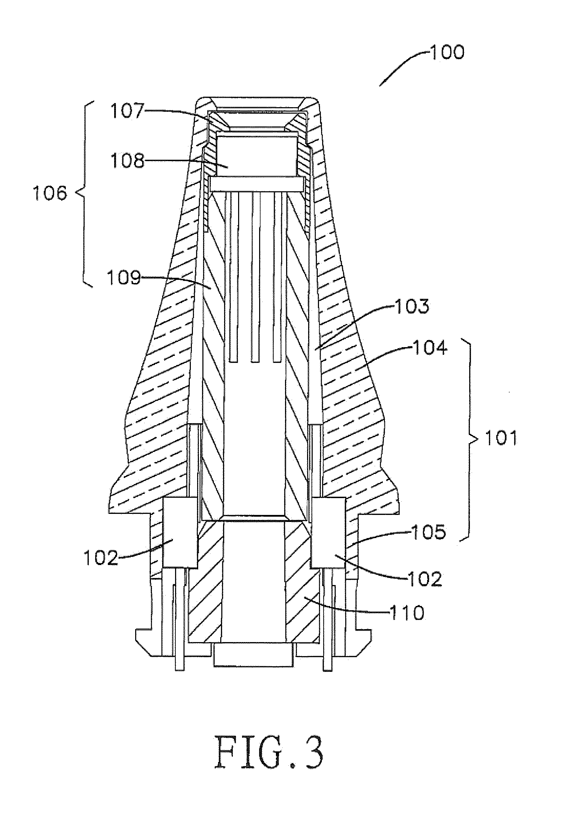

Please refer to Figures 1 to 3, wherein Figure 1 shows a perspective view of a first preferred embodiment of the present invention. Figure 2 shows an explosion view of the first preferred embodiment of the present invention. Figure 3 shows a sectional view of the first preferred embodiment of the present invention.

In the drawings, there is a probe structure 100, and the probe structure 100 includes a light transmissive unit 101, two light sources 102, a temperature detecting module 106, and a base 110.

The light transmissive unit 101 is in open-cylinder shape and has a hollow chamber 103, and the light transmissive unit 101 includes a light transmissive portion 104 and a light transmissive unit base 105. The light transmissive portion 104 is connected to the light transmissive unit base 105. In this embodiment, the light transmissive portion 104 and the light transmissive unit base 105 are integrally formed.

Two light sources 102 are provided in the hollow chamber 103. Each light source 102 can emit one or more colors of lights, such as green and/or red, to and through the light transmissive portion 104 of the light transmissive unit 101. In this embodiment, the light sources 102 are showed by two light emitted diodes. However, the number of the light sources in the present invention is not limited. Further, a person skilled in the art may know that in addition to the color of light may be changed, the power of the light can also be changed according to practical design needs.

The temperature detecting module 106 includes a copper cap 107, a temperature detecting element 108, and a copper sleeve 109. The copper cap 107, temperature detecting element 108, and copper sleeve 109 are assembled in sequence to form the temperature detecting module 106. The temperature detecting module 106 is provided in the hollow chamber 103.

The base 110 is provided in the light transmissive unit base 105 such that the light sources 102 are tightly secured in the hollow chamber 103.

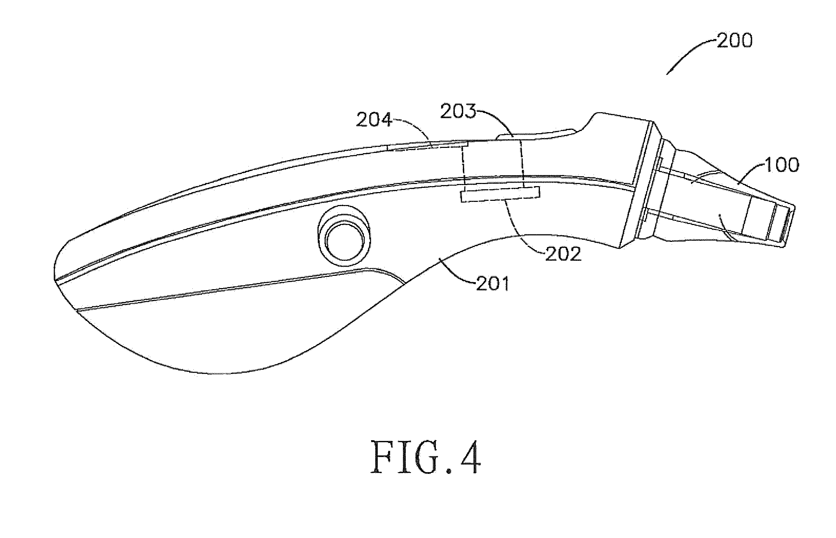

Please refer to Figures 1 to 4, wherein Figure 4 shows a perspective view of a second preferred embodiment of the present invention. That is, Figure 4 shows a temperature measuring device 200 that utilizes the probe structure 100 of Figures 1 to 3. The temperature measuring device 200 is in an ear thermometer form. A person skilled in the art may know that the temperature measuring device can be assembled with a probe structure in any other form, such as a body thermometer form.

In addition to the probe structure 100, the temperature measuring device 200 in the drawings further includes a temperature measuring body 201. The probe structure 100 is assembled in the temperature measuring body 201. Further, the temperature measuring body 201 includes a circuit board 202, a button 203, and a temperature display screen 204.

The button 203 is electrically connected to the circuit board 202, the temperature display screen 204 is electrically connected to the circuit board 202, and the temperature detecting module 106 of the probe structure 100 is also electrically connected to the circuit board 202. It should be noticed that, the button 203 is used to activate the whole circuit board 202 and elements connect thereto, therefore, the button, in a general sense, is a kind of switch. Further, in addition to indicating the value of temperature, the temperature display screen 204 can display different colors of back lights such that a user can know the condition of a subject (such as fever) from the displayed result by displaying the color of back lights.

Therefore, the above-mentioned temperature measuring device 200 can emit one or more colors of lights by the design of light sources 102, and with the light transmissive design of the light transmissive unit 101, users can easily place the probe structure 100 to the site to be measured. In this embodiment, the site to be measured is ear, however, it is not limited in the present invention.

Moreover, if the temperature measured by the temperature measuring device 200 exceeds a predetermined temperature (such as 37.5°C), the light sources 102 may emit one or more colors of lights so that different conditions can be indicated by changing the colors of light. For example, when a subject gets a fever (body temperature is higher than 37.5°C), a user can directly know the condition of the subject (such as fever) from the displayed result since his/her sight line focuses on the light transmissive unit 101.

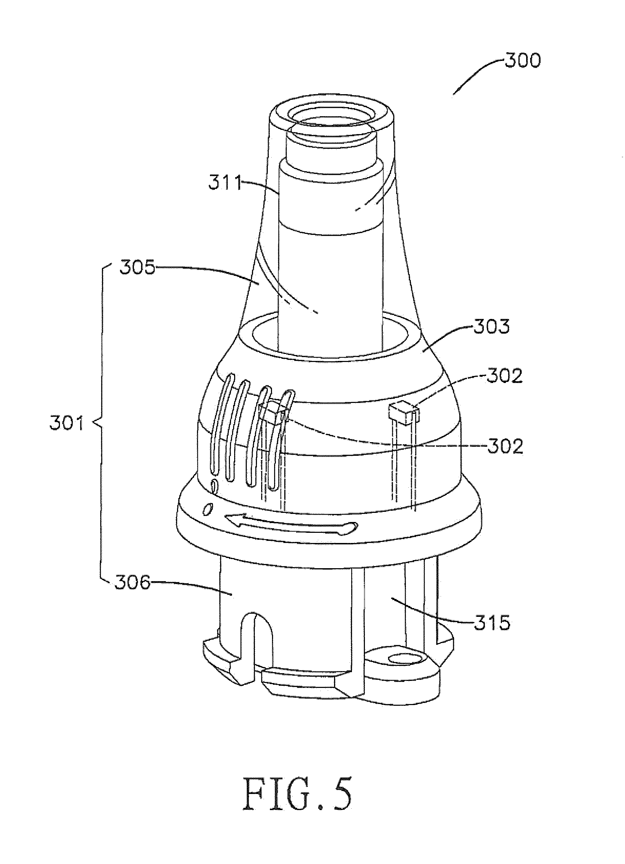

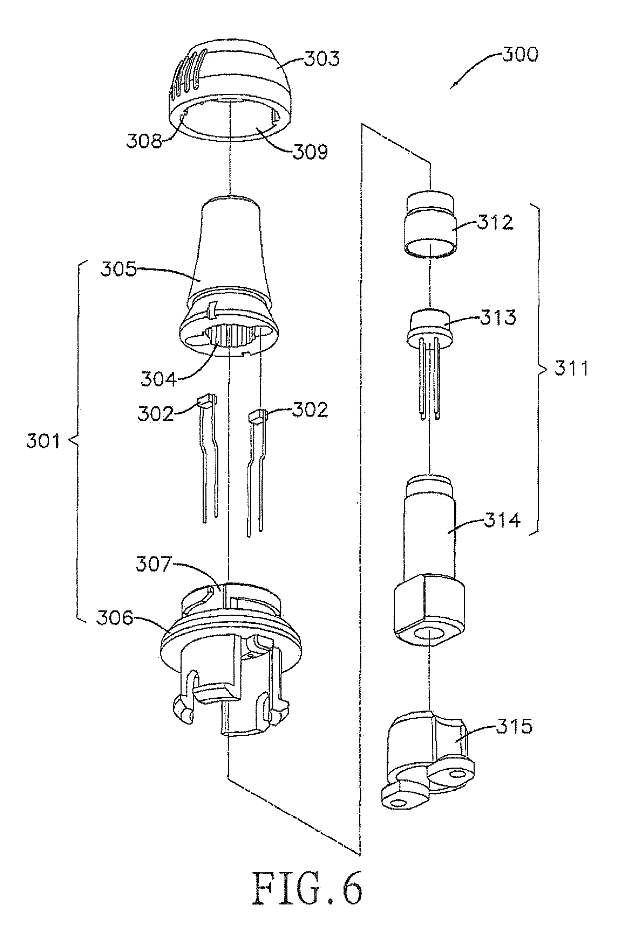

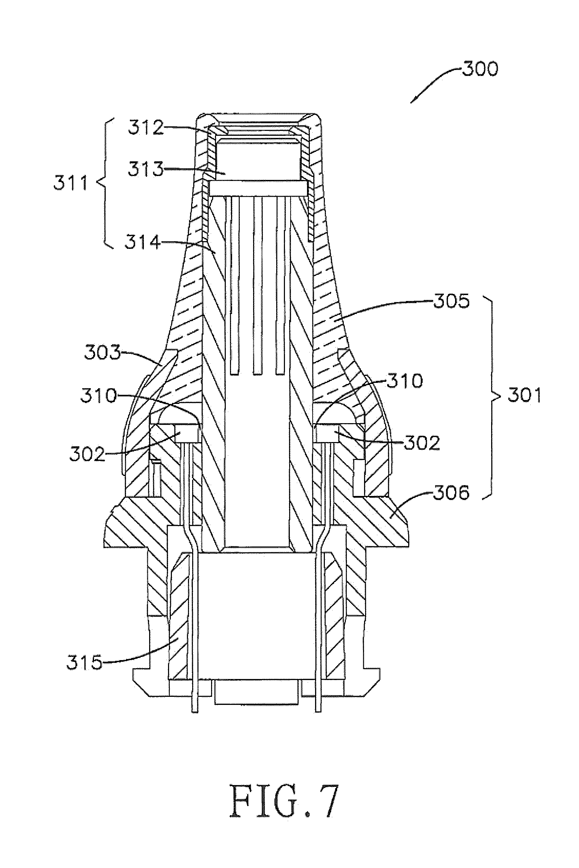

Please refer to Figures 5 to 7, wherein Figure 5 shows a perspective view of a third preferred embodiment of the present invention. Figure 6 shows an explosion view of a third preferred embodiment of the present invention. Figure 7 shows a sectional view of a third preferred embodiment of the present invention.

In the drawings, there is a probe structure 300, and the probe structure 300 includes a light transmissive unit 301, two light sources 302, a probe cover 303, a temperature detecting module 311, and a base 315. In this embodiment, the light sources 302 are light emitting diodes, however, it is not limited in the present invention.

The light transmissive unit 301 is in open-cylinder shape and has a hollow chamber 304, and the light transmissive unit 301 includes a light transmissive portion 305 and a light transmissive unit base 306. The light transmissive unit base 306 includes a groove 307. The probe cover 303 includes a protrusion 308. When the protrusion 308 snaps in the groove 307, the light transmissive portion 305 has contact to the light transmissive unit base 306. In other embodiments, the light transmissive unit base can include a protrusion, the probe cover can include a groove, and the protrusion can snap in the groove. However, a person skilled in the art may understand that the method to secure the probe cover 303 to the light transmissive unit base 306 is not limited in this way and can be modified according to practical designs.

In the drawings, the light transmissive portion 305, the light transmissive unit base 306, and the probe cover 303 are designed to be separate components. In assembling, the light transmissive unit 301 is housed in a second hollow chamber 309 of the probe cover 303 and the light transmissive portion 305 protrudes through the probe cover 303 and exposes outwardly.

Moreover, two light sources 302 are provided in the light transmissive unit base 306. The light sources 302 emit lights to and through the light transmissive portion 305 of the light transmissive unit 301.

In design perspective, the light transmissive unit base 306 of the light transmissive unit 301 can be provided with two recesses 310. Each recess 301 houses one light source 302. Further, a person skilled in the art may know that the number of the recess 310 can be changed according to the number of the light sources 302, which may be changed according to the practical design needs.

The temperature detecting module 311 includes a copper cap 312, a temperature detecting element 313, and a copper sleeve 314. The copper cap 312, temperature detecting element 313, and copper sleeve 314 are assembled in sequence to form the temperature detecting module 311. The temperature detecting module 311 is provided in the first hollow chamber 304.

The base 315 is provided in the light transmissive unit base 306.

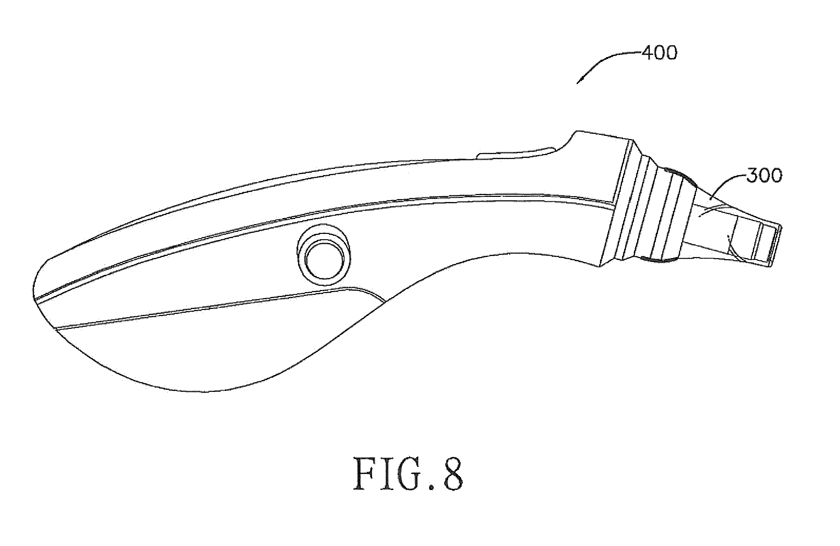

Please refer to Figures 5 to 8, wherein Figure 8 shows a perspective view of a fourth preferred embodiment of the present invention. That is, Figure 8 shows a temperature measuring device 400 that utilizes the probe structure 300 of Figures 5 to 7. The temperature measuring device 400 is in an ear thermometer form. The relevant description can be referred to the second preferred embodiment of Figure 4 and is omitted here.

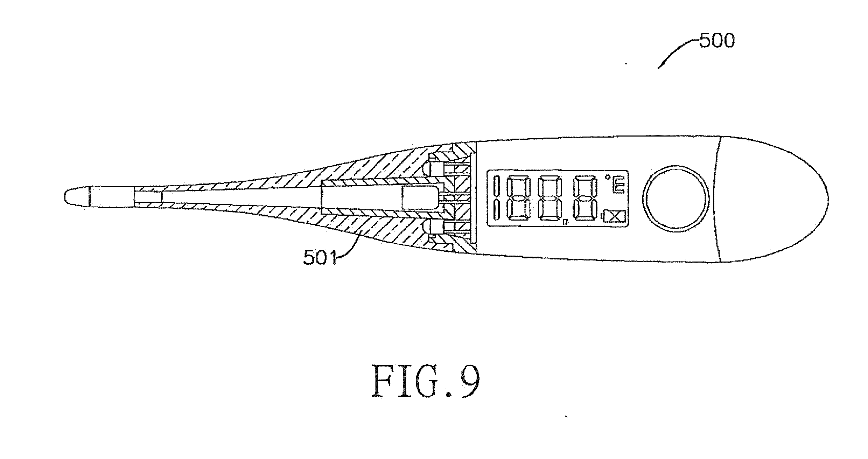

Please refer to Figure 9, wherein Figure 9 shows a partial sectional view of a fifth preferred embodiment of the present invention. In Figure 9, the probe structure 501 of the embodiment is the same as the first preferred embodiment, except the external shape. The temperature measuring device 500 of the embodiment is in a body thermometer form. The relevant description can be referred to the second preferred embodiment of Figure 4 and is omitted here.

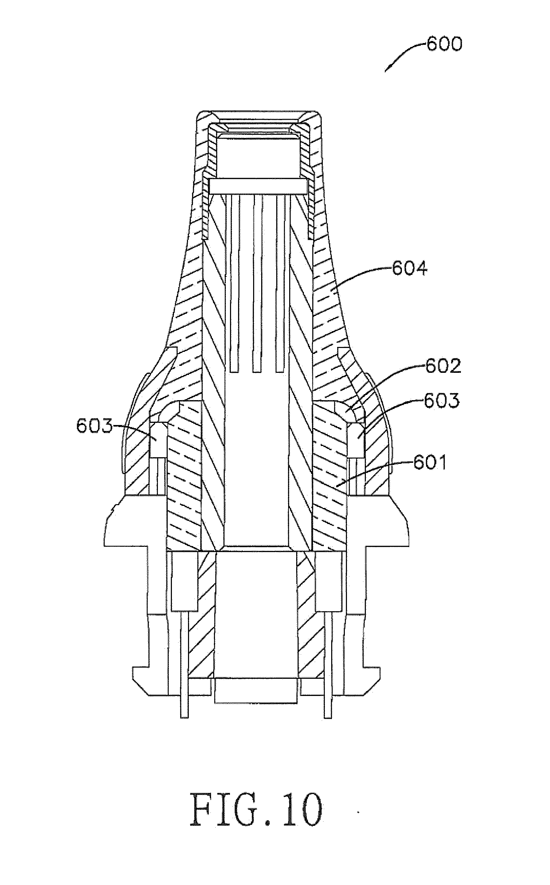

Please refer to Figure 10, wherein Figure 10 shows a sectional view of a sixth preferred embodiment of the present invention. The primary structure of the probe structure 600 of the embodiment is the same as the third preferred embodiment (as shown in Figures 5 to 7), except a light guide plate 601 is provided. The light guide plate 601 is provided in the hollow chamber 602 and is adjacent to the light sources 603 and the light transmissive portion 604. Two light sources 603 emit lights to and through the light transmissive portion 604 via the light guide plate 601.

The method of using the temperature measuring device having the probe structure will be described by corresponding to the probe structure 100 showed in Figures 1 to 3 and the temperature measuring device 200 showed in Figure 4. The temperature measuring device 400 showed in Figure 8 and the temperature measuring device 500 showed in Figure 9 use the same method.

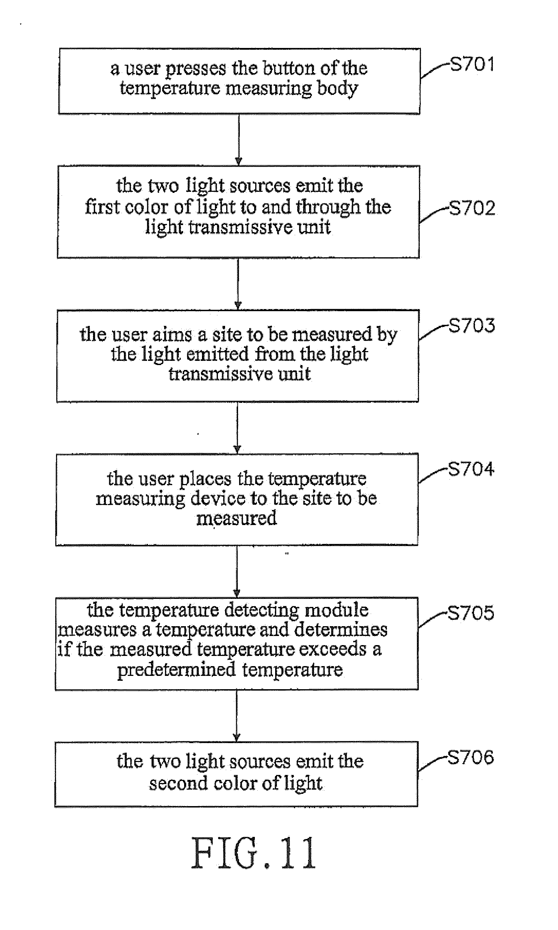

Please refer to Figure 11, wherein Figure 11 shows a flow chart of a seventh preferred embodiment of the present invention. Please also refer to Figures 1 to 4, the related numeral references are represented by Figures 1 to 4.

In this embodiment, two light sources 102 emit a first color (such as green) of light and a second color (such as red) of light. The method of using the temperature measuring device 200 includes the following steps. First, a user presses the button 203 of the temperature measuring body 201 (S701); then light sources 102 emit the first color of light to and through the light transmissive unit 101 (S702). Then, the user aims a site to be measured of a subject (such as ear) by the light emitted from the light transmissive unit 101 (S703); and then the user places the temperature measuring device 200 to the site to be measured (S704). Then, the temperature detecting module 106 measures a temperature and determines if the measured temperature exceeds a predetermined temperature (S705); and finally, the light sources 102 emit the second color of light (S706). In this embodiment, the second color of light includes blink light, however, it is not limited in the present invention.

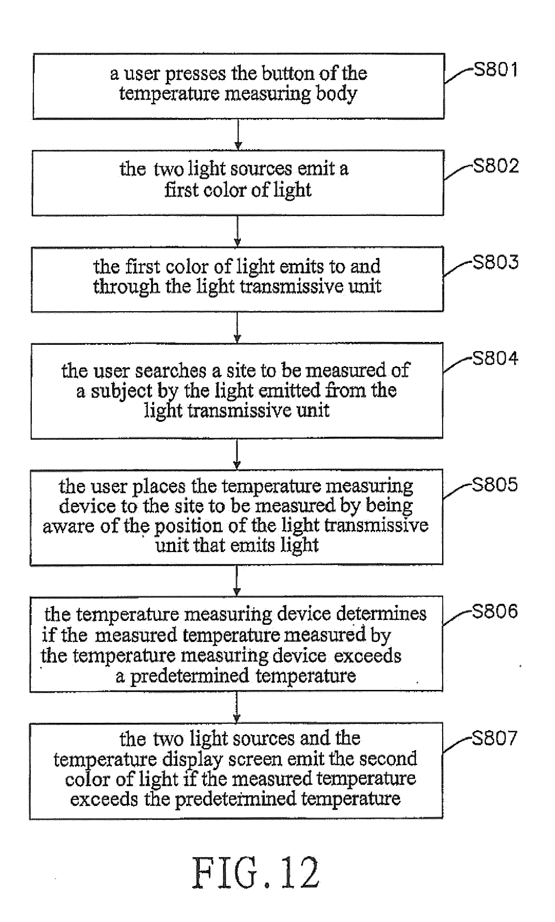

Please refer to Figure 12, wherein Figure 12 shows a flow chart of an eighth preferred embodiment of the present invention. Please also refer to Figures 1 to 4, the related numeral references are represented by Figures 1 to 4.

In this embodiment, users can use the temperature measuring device 200 according to the following steps:
(A) a user presses the button 203 of the temperature measuring body 201 (S801);
(B) the light sources 102 emit a first color of light (S802);
(C) the first color of light emits to and through the light transmissive unit 101 (S803);
(D) the user searches a site to be measured of a subject (such as ear) by the light emitted from the light transmissive unit 101 (S804);
(E) the user places the temperature measuring device 200 to the site to be measured by being aware of the position of the light transmissive unit 101 that emits light (S805);
(F) the temperature measuring device 200 determines if a measured temperature measured by the temperature measuring device 200 exceeds a predetermined temperature (S806); and
(G) the light sources 102 and the temperature display screen 204 emit the second color of light if the measured temperature exceeds the predetermined temperature (S807). In this embodiment, the second color of light includes blink light.

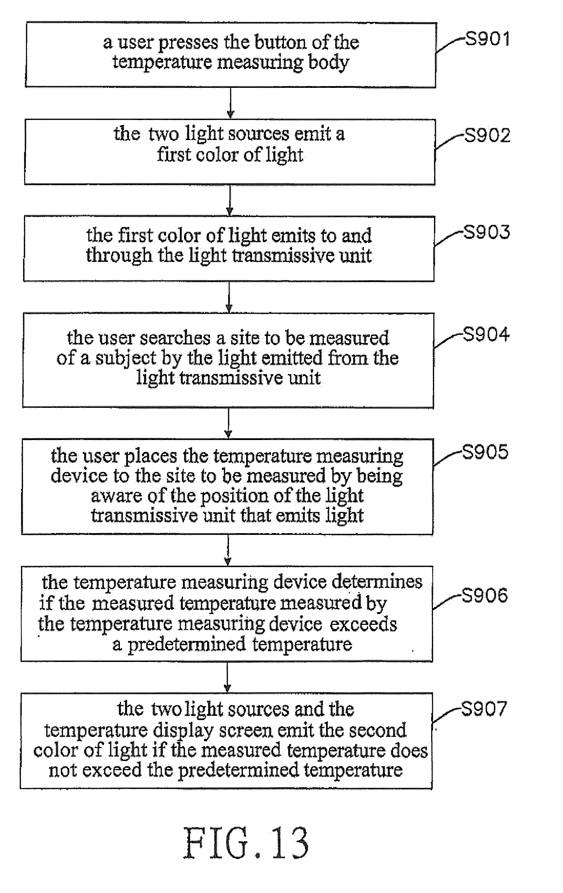

Please refer to Figure 13, wherein Figure 13 shows a flow chart of a ninth preferred embodiment of the present invention, Please also refer to Figures 1 to 4, the related numeral references are represented by Figures 1 to 4.

In this embodiment, users can use the temperature measuring device 200 according to the following steps:
(A) a user presses the button 203 of the temperature measuring body 201 (S901);
(B) the light sources 102 emit a first color of light (S902);
(C) the first color of light emits to and through the light transmissive unit 101 (S903);
(D) the user searches a site to be measured of a subject (such as ear) by the light emitted from the light transmissive unit 101 (S904);
(E) the user places the temperature measuring device 200 to the site to be measured by being aware of the position of the light transmissive unit 101 that emits light (S905);
(F) the temperature measuring device 200 determines if a measured temperature measured by the temperature measuring device 200 exceeds a predetermined temperature (S906); and
(G) the light sources 102 and the temperature display screen 204 emit the second color of light if the measured temperature does not exceed the predetermined temperature (S907). In this embodiment, the second color of light includes blink light.

Therefore, from the above description, when a user wants to use a temperature measuring device 200 in dark environment, he/she can press the button 203 of the temperature measuring body 201 so that the light sources 102 emit the first color of light to and through the light transmissive unit 101. Then, the user can aim the light emitted from the light transmissive unit 101 to the site to be measured, or can search the site to be measured of a subject (such as ear) by the light emitted from the light transmissive unit 101. And then, the user can easily place the temperature measuring device 200 to the site to be measured by being aware of the position of the light transmissive unit 101 that emits light. It should be notice that, a person skilled in the art may know the site to be measured can be forehead, mouth, underarm, anus, and so on when the temperature measuring device is in other form.

After that, the temperature detecting module 106 measures the body temperature. If a measured temperature measured by the temperature detecting module 106 exceeds a predetermined temperature, for example high body temperature when get fever, the light sources 102 may emit a second color of light. At this time, the user can directly and clearly understand this condition by the color change of the light transmissive unit 101. Or, the light sources 102 and the temperature display screen 204 can both emit a second color of light. Alternatively, the light sources 102 and the temperature display screen 204 may also emit a second color of light. Further, the light sources 102 and the temperature display screen 204 may emit a second color of light if a measured temperature measured by the temperature detecting module 106 does not exceed a predetermined temperature. In other words, the design approach of the color change to warn users can be modified according to practical needs.

## Claims

1. A probe structure, comprising:
a light transmissive unit; and
one or more light sources, for emitting one or more colors of lights to and through the light transmissive unit.

2. The probe structure according to claim 1, wherein the light transmissive unit is in open-cylinder shape and has a hollow chamber, the light transmissive unit includes a light transmissive portion and a light transmissive unit base, the light transmissive portion is connected to the light transmissive unit base.

3. The probe structure according to claim 1, further comprising a probe cover, and the light transmissive unit is in open-cylinder shape and has a hollow chamber, and the light transmissive unit includes a light transmissive portion and a light transmissive unit base, the light transmissive unit base includes a groove, the probe cover includes a protrusion, wherein, when the protrusion snaps in the groove, the light transmissive portion has contact to the light transmissive unit base.

4. The probe structure according to claim 2 or 3, wherein the one or more light sources are provided in the light transmissive unit base and emit lights to and through the light transmissive portion.

5. The probe structure according to claim 2 or 3, wherein the one or more light sources are provided in the hollow chamber and emit lights to and through the light transmissive portion.

6. The probe structure according to claim 5, further comprising a light guide plate provided in the hollow chamber, the one or more light sources emit lights to and through the light transmissive portion ofthe light transmissive unit via the light guide plate.

7. The probe structure according to claim 3, wherein the light transmissive unit base includes one or more recesses, each recess houses the one or more light sources.

8. The probe structure according to claim 2 or 3, further comprising a temperature detecting module provided in the hollow chamber.

9. A temperature measuring device having a probe structure, comprising:
the probe structure according to claim 8; and
a temperature measuring body, to which the probe structure being assembled, the temperature measuring body comprising:
a circuit board; and
a button electrically connected to the circuit board;
wherein, the temperature detecting module of the probe structure is electrically connected to the circuit board.

10. The method of using the temperature measuring device according to claim 9, wherein the lights of the one or more light sources include a first color of light and a second color of light, the method comprising the steps of:
(A) pressing the button of the temperature measuring body; and
(B) emitting the first color of light from the one or more light sources to and through the light transmissive unit.

11. The method of using the temperature measuring device according to claim 10, further comprising the following steps after step (B):
(C) aiming the light emitted from the light transmissive unit at a site to be measured;
(D) positioning the temperature measuring device to the site to be measured;
(E) determining if a measured temperature measured by the temperature detecting module exceeds a predetermined temperature; and
(F) emitting the second color of light from the one or more light sources.
